# EUROPEAN PATENT SPECIFICATION

(11) **EP 2 578 157 B1**
(45) Date of publication and mention of the grant of the patent: **19.03.2014**
(21) Application number: 12179032.3
(22) Date of filing: 02.08.2012
(51) Int. Cl.: A61B 6/14

(54) **Device for the acquisition of panoramic radiographies and CBCT volumetric radiographies**
Vorrichtung zur Erfassung von Panorama-Röntgenaufnahmen und DVT-Volumenröntgenaufnahmen
Dispositif d'acquisition de radiographies panoramiques et de radiographies CBCT volumétriques

(30) Priority: 05.10.2011 IT BO20110566
(43) Date of publication of application: 10.04.2013
(73) Proprietor: CEFLA S.C., 40026 Imola (IT)
(72) Inventor: Baldini, Antonio, 40026 Imola (IT); Becca, Antonio, 40026 Imola (IT); Bianconi Davide, 40023 Castel Guelfo di Bologna (IT)
(74) Representative: Herrmann, Franz

(56) References cited:
- EP-A1- 2 156 791
- EP-A2- 1 752 099
- WO-A1-2008/028988
- WO-A1-2010/128404

## Description

The invention relates to an apparatus for dental radiography:
- comprising a support allowing to move an imaging system along predefined trajectories for producing panoramic images and tomographic images of the dentition of a patient,
- the imaging system comprising an X-ray source, and a first X-ray sensor for producing the panoramic images, and a second X-ray sensor for producing the tomographic images,
- the first X-ray sensor and the second X-ray sensor being arranged on an alternating mechanism, which allows making either the first sensor or the second sensor face the X-ray source.

Such an apparatus is known form EP 1 752 099 B1. The known apparatus is a combined panoramic and computed tomography apparatus, and is provided with a sensor for panoramic radiography and a sensor for computer tomography (= CT). Both sensors are mounted on a sensor unit. The sensor unit can be rotated around an excentric axis, which is located at a point between the CT sensor and a central point between the panoramic sensor and the CT sensor, so as to alternatively oppose the panoramic sensor and the CT sensor to an X-ray source.

The present invention thus refers to the technical field of extraoral dental radiography, and particularly to a device alternatively performing panoramic radiographies, cone beam volumetric radiographies of facial skeleton and cranial teleradiographies. All these types of radiographies are well known in the art.

Panoramic radiography (also known as orthopantomography) produces a radiographic image of a curved plan approximating patient jaws, with blurring of the anatomical structures lying outside a narrow layer around the predefined curved plane.

Cone beam volumetric radiography (also known as CBCT) is the acquisition, from different projection angles, of a series of two-dimensional radiographic images which will be processed after the acquisition to reconstruct tridimensional volumes.

Teleradiography is a projective radiographic technique, producing different radiographic projection images of the skull or of other anatomical areas from different points of view, with minimum magnification and geometrical distortion. Usually two perspectives are represented, latero-lateral and anteroposterior.

Two types of equipment are generally available on the market: a first type is panoramic equipment, alternatively performing panoramic radiographies and teleradiographies, and a second type is equipment dedicated to CBCT volumetric radiography. These equipments are expensive and cumbersome.

Proceeding from this related art, the present invention seeks to provide a radiographic apparatus, which is capable of alternatively performing, after an appropriate set-up of the apparatus, said three types of radiologic examination, and which can be easily and cheaply produced.

This object is achieved by a radiographic apparatus having the features of the independent claim. Advantageous embodiments and refinements are specified in claims dependent thereon.

The radiographic apparatus according to the present invention has an X-ray source and different X-ray sensors, of the type appropriate for the examination to be performed, arranged at an appropriate distance from the X-ray source so that good-quality images of high quality can be obtained. The X-ray source is the same for the three types of radiographic acquisition. In particular, the invention consists in the respective disposition of the panoramic (PAN) and the cone beam volumetric radiography (CBCT) sensors, which are respectively arranged at an angle.

In selecting the angle between PAN sensor and CBCT sensor different issues have to be taken into account: set-up speed, temperature, and protection from non-collimated X-rays.

The shortest set-up time of the apparatus for the specific acquisition is desirable so that it will be as rapid as possible: a small angle between the two sensors would accelerate their exchange.

When the sensor is active, and especially when it has to acquire and transmit a great number of signals, as it is needed in this type of application, heat is produced: this would lead to the longest possible distance between PAN sensor and CBCT sensor, in accordance with the object of an efficient heat removal.

When X-rays are emitted from the X-ray source, a small quantity of rays might hit outside the sensor's sensitive area, potentially degrading the gathered signal. The respective disposition of the sensors should be able to avoid this risk.

From all the above-cited considerations, the optimal respective position for the two sensors is an angle ranging between 40° and 140°, preferably between 80° and 100°, most preferably at 90°. This particular disposition allows the X-ray operator to set the apparatus rapidly and conveniently for the patient.

Moreover, the present disposition allows inserting a further element, e.g. an optional collimator or a camera taking pictures of the patient undergoing the radiographic acquisition.

The positions of PAN and CBCT sensors on the alternating mechanism, and therefore the distance of the specific sensor from the X-ray source, are appropriately chosen such as to obtain the best radiographic result. The distance between X-ray source and sensor varies with the specific sensor used.

The apparatus comprises a base from which a post raises, the post supporting a device capable of vertically moving a C-arm, the C-arm carrying on one side the X-ray source and a primary collimator immediately downstream the X-rays exit, and on the other side two different types of sensors. In particular, the PAN sensor and the CBCT sensor are arranged on a mechanism alternating the two sensors, bringing the sensor needed for the desired acquisition in the position where it can be hit by X-rays, and at the same time removing from the X-ray path the undesired sensor. When a teleradiography has to be acquired, the alternating mechanism removes PAN and CBCT sensors from the X-ray path.

Two different configurations of the apparatus are possible: a minimal configuration with a PAN sensor and a CBCT sensor, without teleradiography support. A second optional configuration is a complete radiographic apparatus, alternatively allowing obtaining panoramic radiographies, CBCT volumetric radiographies images and teleradiography images, and therefore also having a support for the teleradiography.

For a radiographic apparatus capable of performing teleradiography, two embodiments are possible: one simpler and cheaper embodiment, in which only a single PAN sensor is present, and this PAN sensor is detached from the PAN support and positioned on the teleradiography support when a teleradiography has to be acquired. In a second, more expensive embodiment, two sensors are present, one PAN sensor and a teleradiography sensor. In this second embodiment, the teleradiography sensor is bigger than the PAN sensor.

The set up of the apparatus can be performed before or after the positioning of the patient in the X-ray apparatus.

Further advantages and properties of the present invention are disclosed in the following description, in which exemplary embodiments of the present invention are explained in detail based on the drawings:
- Figure 1: is a prospective view of the complete radiographic apparatus;
- Figure 2a: is a schematic view of the position of PAN, CBCT and CEPH sensors during a panoramic image acquisition;
- Figure 2b: is a schematic view of the position of PAN, CBCT and CEPH sensors during a CBCT volumetric radiography acquisition;
- Figure 2c: is a schematic view of the position of PAN, CBCT and CEPH sensors during a teleradiography image acquisition;
- Figure 3a: is a perspective view of the mechanism alternating PAN and CBCT sensor;
- Figure 3b: is a horizontal cross-sectional view of the mechanism alternating PAN and CBCT sensor.

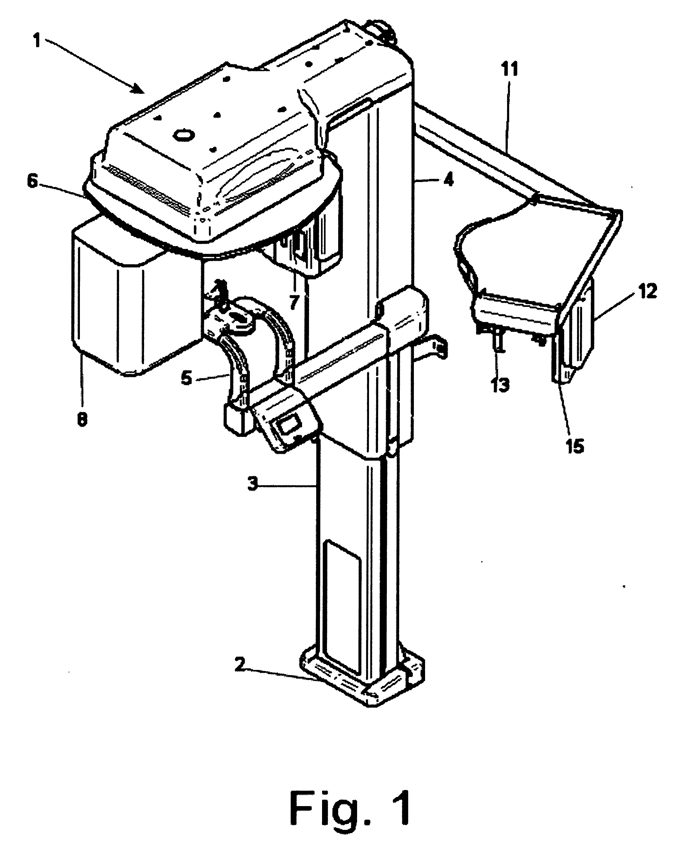

Figure 1 shows in its entirety a radiographic apparatus 1, which can alternatively produce panoramic radiographies, CBCT volumetric radiographies and teleradiography images. The apparatus 1 comprises a base 2 and a post 3 supporting an extension 4, which is provided with a vertical section sliding on the post 3 and a horizontal section. The vertical section of the extension 4 allows the vertical movement of a C-arm 6, which is attached to the horizontal section of the extension 4 and which in its turn supports an X-ray source 7, and an alternating mechanism 8, for alternating the positions of a PAN sensor 9 and a CBCT sensor 10. Moreover, a device 5 for the positioning of the patient is present. On a further optional arm 11, arranged on support 4, a support 12 for CEPH sensor 15 for teleradiography and a further device 13 for positioning the patient are present.

In all acquisition modalities, the X-rays must impinge on the specific sensor: for radiography acquisition the sensor must be brought in an appropriate position allowing the X-rays are to impinge on the dedicated sensor.

C-arm 6 has the X-ray source 7 at one of its ends, and at its other end, the alternating mechanism 8, on which PAN sensor 9 and CBCT sensor 10 are disposed. The alternating mechanism 8 is fixed to C-arm 6 so that it can be moved either manually or automatically exposing the specific sensor needed to the X-ray source 7.

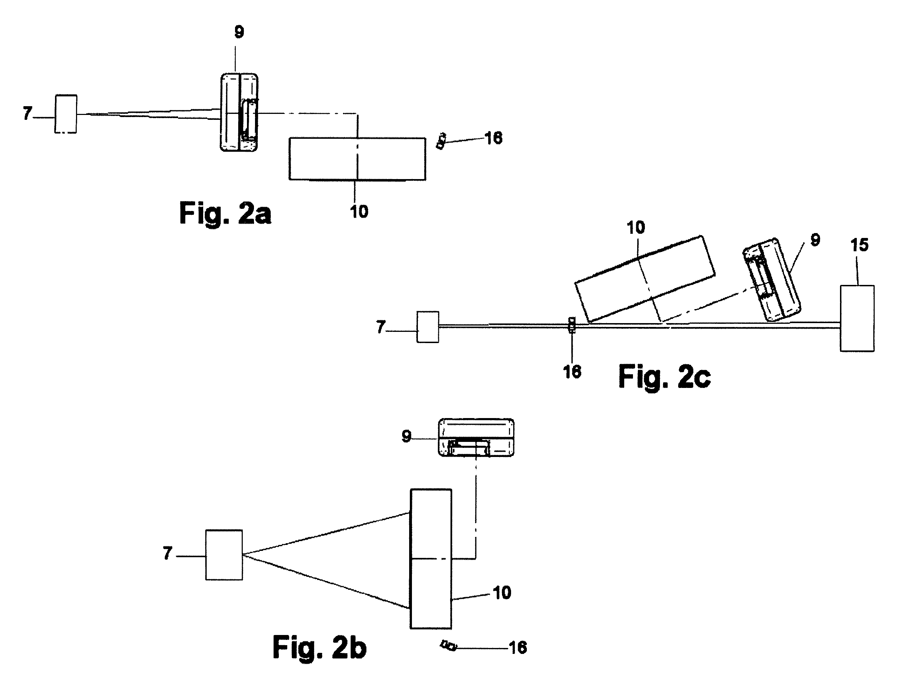

The alternating mechanism 8 has at least two stops, schematically shown in Figures 2a, 2b, 2c: one stop bringing the PAN sensor 9 in the position needed for the acquisition of panoramic images, and another stop bringing the CBCT sensor 10 in the position needed for the acquisition of CBCT volumetric radiographies. The acquisition can start only when the specific sensor is in the proper stop position. The sensor remains in its stop position during the whole specific acquisition process, and can only be moved after the specific acquisition process has terminated.

The positions of PAN sensor 9 and CBCT sensor 10 on alternating mechanism 8, and therefore the distance of the specific sensor from the X-ray source 7 are chosen such as to get the best radiographic result. The distance between X-ray source 7 and specific sensor varies with the type of sensor used. The distance between PAN sensor 9 and X-ray source 7 is generally shorter than the distance between CBCT sensor 10 and X-ray source 7.

In accordance with the magnification degree and the consequent spatial distortion which are held optimal for the kind of acquisition, alternating mechanism 8 is arranged such that it can bring PAN sensor 9 for the panoramic acquisition at a distance usually ranging between 520 and 580 mm from X-ray source 7, while CBCT sensor 10 in CBCT volumetric radiography is positioned at distances usually ranging between 600 and 700 mm from X-ray source 7.

In cases where the machine comprises an optional secondary collimator 16 mounted on the alternating mechanism 8 for beam collimation during the teleradiographic acquisition, for a good alignment, the distance between X-ray source 7 and CEPH sensor 15 ranges between 1400 and 1800 mm.

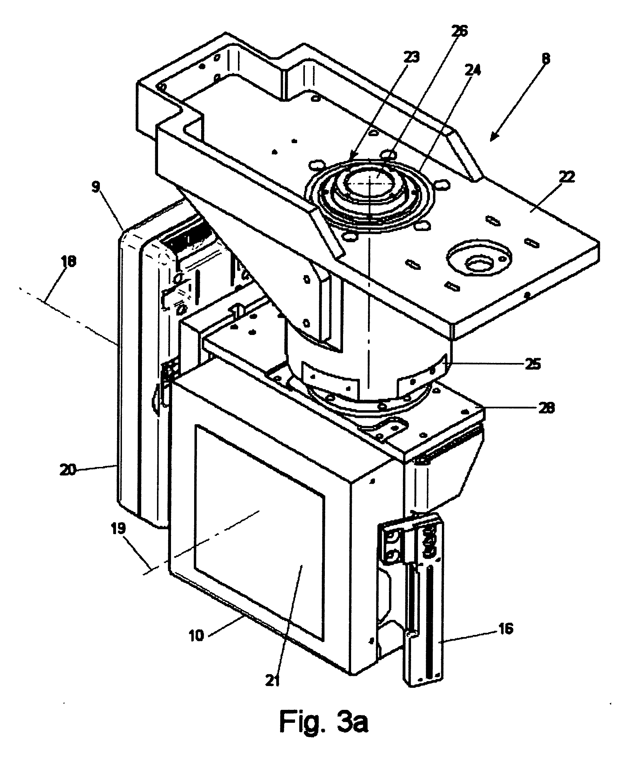

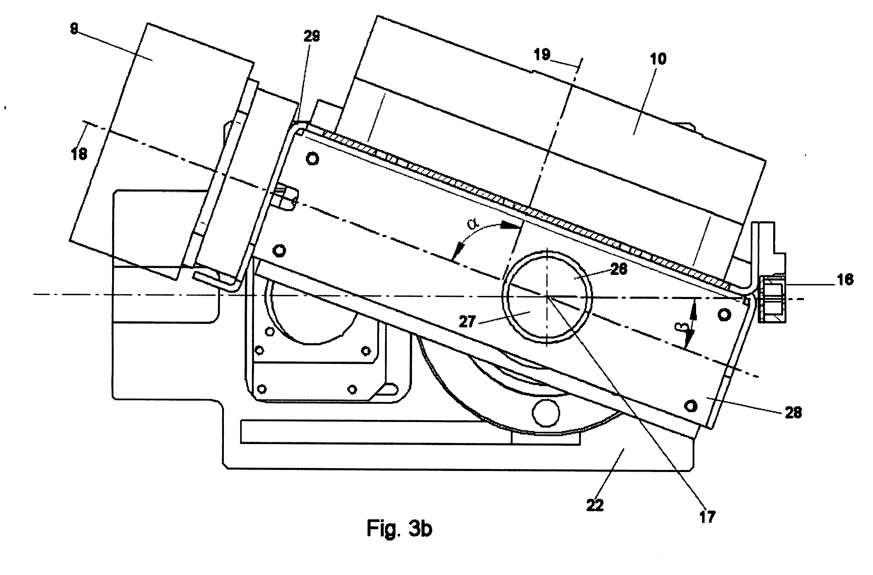

Keeping in mind these dimensions, a preferred arrangement of the elements in the alternating mechanism 8 according to the objects of the present invention, in particular with the object of optimizing compactness and setup speed, is shown in Figures 3a and 3b.

Figure 3a is a perspective view of the alternating mechanism 8, by which the PAN sensor 9, the CBCT sensor 10, and the secondary collimator 16 can be pivoted around a rotation axis 17. Figure 3b is a cross-sectional view of the alternating mechanism 8. Figure 3a and 3b further show a PAN sensor axis 18 and CBCT sensor axis 19. The PAN sensor axis 18 and the CBCT sensor axis 19 are centered normals to a sensitive area 20 of the PAN sensor 9 and to a sensitive area 21 of the CBCT sensor 10. In the embodiment depicted in Figure 3a and 3b, the PAN sensor axis 18 and the CBCT sensor axis 19 are positioned at an angle α of 90°, respectively; the distance of the photosensitive area 20 of PAN sensor 9 ranges between 220 and 180 mm from the rotation centre 17 of alternating mechanism 8, the distance between the photosensitive area 21 of CBCT sensor 10 and rotation centre 17 ranges between 50 and 130 mm; finally, secondary collimator 16 is at a distance between 80 and 120 mm from the rotation centre 17 of mechanism 8 and positioned at an angle β ranging between 15° and 25° with respect to the PAN sensor axis 18 of PAN sensor 9. The figures show a preferred embodiment wherein the secondary collimator 16 is placed near the CBCT sensor 10, but this placement is only one of the many possible embodiments.

The alternating mechanism 8 may comprises a plate 22, which can be fixed to the arc 6. The plate 22 is provided with a central opening 23, in which a reduced section 24 of a stepper motor 25 is fixed. If the alternating mechanism 8 is mounted on the arc 6, a drive shaft 26 is oriented in a vertical direction. At the other end 27 of the drive shaft 26, a plate 28 with a supporting mechanism 29 of the various elements (PAN sensor 9, CBCT sensor 10 and secondary collimator 16) is mounted. The stepper motor 25 can be electronically actuated by a microcontroller. The stepper motor 25 is driven by a drive circuit, when the microcontroller sends a set-up command (for PAN, CBCT or CEPH acquisitions) to the drive circuit. Stop positions can be detected with photoelectric cells, one for each stop position, so that the passage from one configuration to the other can occur without passing from an initial position, and the need for counting motor steps for getting to the desired positions.

The mechanisms for sensors mounting/demounting can also be provided on the plate 28 (in the case of an apparatus with removable sensors). The plate 28 can also be provided with a mechanism for a vertical positioning of the secondary collimator 16 for an accurate centring of X-ray beam on CEPH sensor 15.

Alternating mechanism 8 is shown in more detail in Figure 3b, which represents a horizontal cross-sectional view of the mechanism 8 itself supporting PAN sensor 9, CBCT sensor 10 and optional secondary collimator 16, respectively. Figure 3b allows recognizing the spatial relationship between the two sensors. Figure 3b shows, in particular, the rotation axis 17 of the alternating mechanism 8.

When a panoramic acquisition has to be performed, the PAN sensor 9 must be brought on the X-ray path and the primary collimator must be set in an appropriate manner. The PAN sensor 9 can be a linear sensor (one-dimensional), well known in the art, but also an area sensor (two-dimensional). The patient is positioned within the apparatus 1 using patient positioning device 5. Once the patient is properly positioned in the apparatus 1, the acquisition can start with the emission of X-rays from X-ray source 7.

When a CBCT volumetric radiography has to be performed, the CBCT sensor 10 must be brought on the X-ray path and the primary collimator must be set in an appropriate manner. The alternating mechanism 8 rotates, manually or automatically, and the CBCT sensor 10 is brought in a position to intercepts X-rays.

In a particular embodiment, the X-rays emission modality can be modified according to the radiographic acquisition: panoramic acquisitions might be performed with an X-ray continuous emission, while CBCT volumetric radiography might be performed with pulsed X-ray emission, in order to get the best radiographic result with the minimal X-ray dose to the patient.

When a teleradiography acquisition must be performed, the anatomic portion of patient to be imaged must be positioned within support 13, and, if the machine is provided with a removable sensor only, PAN sensor 9 must be removed from alternating mechanism 8 and positioned on teleradiography support 12, while the primary collimator and the optional secondary collimator 16 must be set in an appropriate manner. In a more complete embodiment, wherein the apparatus has two sensors 9 and 15, the apparatus is ready for the teleradiography acquisition right after patient positioning.

CEPH sensor 15 can be a linear sensor (one-dimensional) or an area sensor (two-dimensional).

In both cases, alternating mechanism 8 must be positioned so that both PAN sensor 9 and CBCT sensor 10 are excluded from the X-ray path.

On alternating mechanism 8, moreover, a secondary collimator 16 may be provided, which must be arranged such as to be crossed by X-rays, therefore stopping the alternating mechanism 8 in a third position. In this case, too, the acquisition can start only when alternating mechanism 8 is in the proper position. This movement can be performed either manually or automatically. Secondary collimator 16 collimates X-rays during teleradiography acquisition, so that the X-ray hits the sensitive part of the CEPH sensor 15 as precisely as possible.

Once the apparatus 1 is properly set up, teleradiography acquisition can start.

Throughout the description and claims of this specification, the singular encompasses the plural unless the context otherwise requires. In particular, where the indefinite article is used, the specification is to be understood as contemplating plurality as well as singularity, unless the context requires otherwise.

## Claims

1. An apparatus for dental radiography:
- comprising a support (2, 3, 4, 6) allowing to move an imaging system along predefined trajectories for producing panoramic images and tomographic images of the dentition of a patient,
- the imaging system comprising an X-ray source (7), and a first X-ray sensor (9) for producing the panoramic images, and a second X-ray sensor (10) for producing the tomographic images
- the first X-ray sensor (9) and the second X-ray sensor (10) being arranged on an alternating mechanism (8), which allows making either the first sensor (9) or the second sensor (10) face the X-ray source (7),
**characterized in that**
- the alternating mechanism (8) brings either a first sensor axis (18) of the first sensor (9) or a second sensor axis (19) of the second sensor (10) towards the X-ray radiation source (7) by allowing a pivoting motion around an alternation axis (17), and that
- the first sensor axis (18) and the second sensor axis (19) intersect at an angle (α) between 40° and 140°, if the first sensor axis (18) and the second sensor axis (19) are projected along the alternation axis (17) on a plane perpendicular to the alternation axis (17).

2. The apparatus according to claim 1,
wherein the support comprises a base (2) and a post (3), which holds an vertically adjustable extension (4), on which an arm (6) is rotably mounted, one end of the arm (6) being provided with the X-ray source (7) and the other end of the arm (6) being provided with the alternating mechanism (8) holding the first sensor (9) and the second sensor (10).

3. The apparatus according to claim 1 or 2, comprising a further support (11) and a device (13) for positioning a patient during a teleradiography acquisition.

4. The apparatus according to any one of claim 1 to 3, wherein the first X-ray sensor (9) for panoramic imaging is relocable between a position on the alternating mechanism (8) and a position on the further support (11) for teleradiography and is mounted in both positions in a manually detachable way or wherein a third X-ray sensor (15) is arranged on the further support (12) for teleradiography.

5. The apparatus according to any one of claims 1 or 4, wherein the X-ray sensors (9, 10, 15) are linear sensors or area sensors.

6. The apparatus according to any one of claims 1 to 5, wherein the first sensor (9) is at a distance ranging between 520 and 580 mm from the X-ray source (7) during panoramic imaging, while the second sensor (10) for tomographic imaging is at a distance ranging between 600 and 700 mm from the X-ray source (7) during tomographic imaging.

7. The apparatus according to any one of claims 1 to 6, wherein the alternating mechanism (8) further supports a secondary collimator (16).

8. The apparatus according to claim 7, wherein the secondary collimator (16), mounted on the alternating mechanism (8) for the collimation of the X-ray beam, is at a distance ranging between 1400 and 1800 mm from the X-ray source (7) during teleradiography acquisition.

9. The apparatus according to any one of claims 1 to 8, wherein the tomographic imaging is CBCT volumetric radiography.

10. The apparatus according to any one of claims 1 to 9, wherein a camera taking pictures of the patient undergoing the radiographic acquisition is arranged on the alternating mechanism (8).

11. The apparatus according to any one of claims 1 to 10, wherein the first sensor axis (18) and the second sensor axis (19) intersect at an angle (α) between 80° and 100°.

12. The apparatus according to any one of claims 1 to 11, wherein the first sensor axis (18) and the second sensor axis (19) intersect at an angle (α) of 90°.

## Patentansprüche

1. Vorrichtung für die dentale Radiographie:
- die eine Halterung (2, 3, 4, 6) aufweist, die es gestattet, ein Bildsystem entlang einer vorbestimmten Trajektorie zu bewegen, um Panoramabilder und tomographische Bilder des Gebisses eines Patienten zu erzeugen,
- wobei das Bildsystem eine Röntgenquelle (7) und einen ersten Röntgensensor (9) zum Erzeugen der Panoramabilder und einen zweiten Röntgensensor (10) zum Erzeugen der tomographischen Bilder aufweist,
- wobei der erste Röntgensensor (9) und der zweite Röntgensensor (10) auf einem Wechselmechanismus (8) angeordnet sind, der entweder den ersten Sensor (9) oder den zweiten Sensor (10) der Röntgenquelle (7) zugewandt sein lässt,
**dadurch gekennzeichnet, dass**
- der Wechselmechanismus (8) entweder eine erste Sensorachse (18) des ersten Sensors (9) oder eine zweite Sensorachse (19) des zweiten Sensors (10) zur Röntgenquelle (7) bringt, indem eine Verschwenkbewegung um eine Wechselachse (17) zugelassen wird, und dass
- die ersten Sensorachse (18) und die zweite Sensorachse (19) sich in einem Winkel (α) zwischen 40° und 140° schneiden, wenn die erste Sensorachse (18) und die zweite Sensorachse (19) entlang der Wechselachse (17) auf eine zur Wechselachse (17) rechtwinklige Ebene projiziert sind.

2. Vorrichtung nach Anspruch 1,
bei der die Halterung eine Basis (2) und eine Strebe (3) aufweist, die einen in senkrechte Richtung einstellbaren Ausleger (4) aufweist, an dem ein Arm (6) drehbar angebracht ist, wobei ein Ende des Arms (6) mit einer Röntgenquelle (7) versehen ist und das andere Ende des Arms (6) mit dem Wechselmechanismus (8) versehen ist, der den ersten Sensor (9) und den zweiten Sensor (10) hält.

3. Vorrichtung nach Anspruch 1 oder 2,
die eine weitere Halterung (11) und ein Gerät (13) zum Positionieren eines Patienten während der Aufnahme einer Teleradiographie aufweist.

4. Vorrichtung nach einem der Ansprüche 1 bis 3,
bei der der ersten Röntgensensor (9) für Panoramabildaufnahmen zwischen einer Position auf dem Wechselmechanismus (8) und einer Position auf der weiteren Halterung (11) für die Teleradiographie versetzbar ist und in beiden Positionen in einer von Hand lösbaren Weise angebracht ist oder bei der ein dritter Röntgensensor (15) auf der weiteren Halterung (12) für die Teleradiographie angebracht ist.

5. Vorrichtung nach einem der Ansprüche 1 oder 4,
bei der die Röntgensensoren (9, 10, 15) lineare Sensoren oder Flächensensoren sind.

6. Vorrichtung nach einem der Ansprüche 1 bis 5,
bei der sich der erste Sensor (9) während einer Panoramabildaufnahme in einer Entfernung zwischen 520 und 580 mm von der Röntgenquelle (7) befindet, wohingegen sich der zweite Sensor (10) für tomographische Bildaufnahmen während einer tomographischen Bildaufnahme in einer Entfernung zwischen 600 und 700 mm von der Röntgenquelle (7) befindet.

7. Vorrichtung nach einem der Ansprüche 1 bis 6,
bei der der Wechselmechanismus (8) des Weiteren einen sekundären Kollimator (16) hält.

8. Vorrichtung nach Anspruch 7,
bei der sich der sekundäre Kollimator (16), der für die Kollimation des Röntgenstrahls auf dem Wechselmechanismus (8) angeordnet ist, während einer teleradiographischen Bildaufnahme in einer Entfernung zwischen 1400 und 1800 mm von der Röntgenquelle (7) befindet.

9. Vorrichtung nach einem der Ansprüche 1 bis 8,
bei der die tomographische Bildaufnahme eine CBCT Volumenradiographie ist.

10. Vorrichtung nach einem der Ansprüche 1 bis 9,
bei der eine Kamera, die Bilder von dem sich einer radiographischen Bildaufnahme unterziehenden Patienten aufnimmt, auf dem Wechselmechanismus (8) angeordnet ist.

11. Vorrichtung nach einem der Ansprüche 1 bis 10,
bei der die erste Sensorachse (18) und die zweite Sensorachse (19) sich in einem Winkel (α) zwischen 80° und 100° schneiden.

12. Vorrichtung nach einem der Ansprüche 1 bis 11,
bei der sich die erste Sensorachse (18) und die zweite Sensorachse (19) in einem Winkel (α) von 90° schneiden.

## Revendications

1. Un appareil de radiographie dentaire :
- comprenant un support (2, 3, 4, 6) permettant de déplacer un système d'imagerie le long de trajectoires prédéfinies de façon à produire des images panoramiques et des images tomographiques de la dentition d'un patient,
- le système d'imagerie comprenant une source de rayons x (7) et un premier capteur de rayons x (9) destiné à produire les images panoramiques, et un deuxième capteur de rayons x (10) destiné à produire les images tomographiques,
- le premier capteur de rayons x (9) et le deuxième capteur de rayons x (10) étant agencés sur un mécanisme alternant (8) qui permet soit au premier capteur (9) ou au deuxième capteur (10) de faire face à la source de rayons x (7),
**caractérisé en ce que**
- le mécanisme alternant (8) place soit un premier axe de capteur (18) du premier capteur (9) ou un deuxième axe de capteur (19) du deuxième capteur (10) vers la source de rayonnement à rayons x (7) en permettant un mouvement de pivotement autour d'un axe d'alternance (17), et **en ce que**
- le premier axe de capteur (18) et le deuxième axe de capteur (19) se coupent à un angle (α) entre 40° et 140° si le premier axe de capteur (18) et le deuxième axe de capteur (19) sont projetés le long de l'axe d'alternance (17) sur un plan perpendiculaire à l'axe d'alternance (17).

2. L'appareil selon la Revendication 1,
dans lequel le support comprend une base (2) et un montant (3), qui est muni d'une extension ajustable verticalement (4), sur lequel un bras (6) est monté rotatif, une extrémité du bras (6) étant équipée de la source de rayons x (7) et l'autre extrémité du bras (6) étant équipée du mécanisme alternant (8) comprenant le premier capteur (9) et le deuxième capteur (10).

3. L'appareil selon la Revendication 1 ou 2,
comprenant un autre support (11) et un dispositif (13) destinés au positionnement d'un patient au cours d'une acquisition de téléradiographie.

4. L'appareil selon l'une quelconque des Revendications 1 à 3,
dans lequel le premier capteur de rayons x (9) destiné à une imagerie panoramique est repositionnable entre une position sur le mécanisme alternant (8) et une position sur l'autre support (11) pour une téléradiographie et est monté dans les deux positions d'une manière manuellement détachable ou dans lequel un troisième capteur de rayons x (15) est agencé sur l'autre support (12) pour une téléradiographie.

5. L'appareil selon l'une quelconque des Revendications 1 ou 4,
dans lequel les capteurs de rayons x (9, 10, 15) sont des capteurs linéaires ou des capteurs de secteur.

6. L'appareil selon l'une quelconque des Revendications 1 à 5,
dans lequel le premier capteur (9) se situe à une distance entre 520 et 580 mm de la source de rayons x (7) au cours d'une imagerie panoramique, tandis que le deuxième capteur (10) pour une imagerie tomographique se situe à une distance entre 600 et 700 mm de la source de rayons x (7) au cours d'une imagerie tomographique.

7. L'appareil selon l'une quelconque des Revendications 1 à 6,
dans lequel le mécanisme alternant (8) comprend en outre un collimateur secondaire (16).

8. L'appareil selon la Revendication 7,
dans lequel le collimateur secondaire (16), monté sur le mécanisme alternant (8) pour la collimation du faisceau de rayons x, se situe à une distance entre 1400 et 1800 mm de la source de rayons x (7) au cours d'une acquisition de téléradiographie.

9. L'appareil selon l'une quelconque des Revendications 1 à 8,
dans lequel l'imagerie tomographique est une radiographie volumétrique CBCT.

10. L'appareil selon l'une quelconque des Revendications 1 à 9,
dans lequel un appareil photo prenant des photos du patient faisant l'objet de l'acquisition radiographique est agencé sur le mécanisme alternant (8).

11. L'appareil selon l'une quelconque des Revendications 1 à 10,
dans lequel le premier axe de capteur (18) et le deuxième axe de capteur (19) se coupent à un angle (α) entre 80° et 100°.

12. L'appareil selon l'une quelconque des Revendications 1 à 11,
dans lequel le premier axe de capteur (18) et le deuxième axe de capteur (19) se coupent à un angle (α) de 90°.
